# EUROPEAN PATENT APPLICATION

(11) **EP 4 647 117 A1**
(43) Date of publication of application: **12.11.2025**
(21) Application number: 24174250.1
(22) Date of filing: 06.05.2024
(51) Int. Cl.: A61N 1/375

(54) **GRIPPING AND POSITIONING AID ON A HOUSING OF AN IMPLANT MANUFACTURED BY SELECTIVE LASER MELTING (SLM)**

(71) Applicant: BIOTRONIK SE & Co. KG, 12359 Berlin (DE)
(72) Inventor: Apel, Oliver, 13507 Berlin (DE); Rundfeldt, Martin, 12045 Berlin (DE)
(74) Representative: Biotronik Corporate Services SE

(57) **Abstract**

The present invention relates to an implantable medical device (1), comprising a housing (10) for accommodating at least one component of the implantable medical device (1), and a connection member (13) integrally connected to the housing (10) and protruding from an outside of the housing (10), wherein, the connection member (13) and the housing (10) are integrally formed in one piece out of a metal by selective laser melting (SLM).

## Description

The present invention relates to an implantable medical device, particularly an implantable leadless pacemaker (iLP), as well as to a method for producing a housing of such an implantable medical device.

Implantable medical devices are usually implanted using a delivery system that typically comprises a delivery catheter which may be advanced into a patient to implant the medical device, such as for example an implantable intracardiac pacemaker, in a patient, in particular within the heart of the patient. By means of the delivery catheter an implant location is accessed while the implantable medical device to be implanted is connected to the delivery catheter, and by releasing the implantable medical device from the delivery catheter the implantable medical device may remain at its implant location while the delivery catheter is removed from the patient.

Particularly, implantable intracardiac pacemakers, in contrast to pacemakers implanted subcutaneously using leads extending transvenously into the heart, avoid leads in that the pacemaker device itself is implanted into the heart, the pacemaker having in particular the shape of a capsule for implantation into cardiac tissue, in particular the right ventricular wall of the right ventricle. Such implantable leadless pacemakers offer the advantage of not using electrode leads, which can reduce risks for the patient involved with leads transvenously accessing the heart. Implantable leadless pacemakers may specifically be designed for implantation in the right ventricle and, in this case, during the implantation procedure are placed in or on the right ventricular wall.

The implanting of for example an implantable leadless pacemaker to a specific location of the patient's heart requires proper positioning as well as proper anchoring such that the implantable leadless pacemaker device remains in position when implanted. To provide for an anchoring a leadless pacemaker device may for example comprise an arrangement of anchoring members (such as tines) which may engage with cardiac tissue such that the leadless pacemaker device may be fixated with respect to tissue by means of the anchoring members.

During the process of implantation, the implantable medical device, particularly in form of an implantable intracardiac pacemaker, may be connected to the delivery catheter via a tether that is in turn connected to a housing of the implantable medical device via a connection member (so-called hitch) connected to the housing. As long as the tether is connected to the connection member, the implantable medical implant can in principle be repositioned at the implantation site or even withdrawn into the delivery system.

Currently subtractive manufacturing is used to form housings of implantable leadless pacemakers including the hitch from one piece of titanium or a titanium alloy.

However, subtractive manufacturing of the housings is usually relatively expensive and time consuming. Furthermore, additive manufacturing of the complete housing raises questions regarding the chemical compatibility of the titanium used in additive manufacturing with the battery acid. Furthermore, porosity and gas tightness of the housing might be an issue.

Based on the above, the problem to be solved by the present invention is to provide an implantable medical device allowing a cost-effective manufacturing of its housing, particularly of the connection member connected thereto.

This problem is solved by an implantable medical device having the features of claim 1 as well as by a method having the features of claim 14. Preferred embodiments of these aspects of the present invention are stated in the corresponding dependent claims and are described below.

According to claim 1, an implantable medical device is disclosed, comprising
- a housing for accommodating at least one component of the implantable medical device, and
- a connection member connected to the housing and protruding from an outside of the housing,
wherein,
the connection member is formed on an outside of the housing by selective laser melting.

Particularly, according to a preferred embodiment of the present invention, the housing is configured to accommodate an electronic circuit and/or a battery.

Particularly, the SLM process is used to build the connection member (also denoted as hitch) on the outside of an e.g., existing housing of an implantable medical device (e.g. implantable leadless pacemaker). The housing may thus be manufactured e.g. conventionally and cost-effectively without an additive manufacturing technique such as selective laser melting (SLM) while the more complicated geometry of the connection member is manufactured additively via SLM. The same process may also be used to manufacture other geometries (armature, positioning aids).

Particularly, selective Laser Melting (SLM) is an additive manufacturing (3D printing) technique used to create complex and precise metal parts. Particularly, it is a powder bed fusion process that involves selectively melting metal powder layers using a high-powered laser. In SLM, a thin layer of metal powder is spread onto a build platform or an existing body such as a housing arranged on the building platform, and a laser beam is precisely directed onto the powder to selectively melt it according to a 3D model. The melted powder solidifies to form a solid layer, and the process is repeated by adding new layers of powder and selectively melting them until the complete connection member is built layer by layer.

SLM allows for the fabrication of parts with high precision and intricate internal structures. Selective laser melting is able to process a variety of alloys, allowing prototypes to be functional hardware made out of the same material as production components. Since the components are built layer by layer, it is possible to design complex freeform geometries, internal features and challenging internal passages that could not be produced using conventional manufacturing techniques such as casting or otherwise machined. SLM produces fully dense durable metal parts.

Particularly, selectively melting the powder is accomplished with a high-power laser beam, particularly an ytterbium fiber laser having a power in the range of 2 W to 300 W. The laser beam may be directed in the X and Y directions with two high frequency scanning mirrors and remains in focus along the layer utilizing, e.g., an F-Theta lens arrangement. The laser energy is intense and focused enough to permit full melting (fusion) of the particles to form a solid structure. New powder layers can be evenly spread across the build platform / existing layers by means of a blade or a roller. Parts are built up additively layer by layer, typically using layers of 30 to 60 micrometers thickness.

According to a preferred embodiment of the implantable medical device of the present invention, the connection member is configured to be connected to a tether.

According to yet another preferred embodiment of the implantable medical device, the connection member comprises an opening (through which the tether extends when being connected to the connection member).

Further, according to a preferred embodiment of the implantable medical device, the connection member comprises a rounded surface delimiting the opening for contacting the tether when the tether is received in the opening and extends along said surface.

Furthermore, according to a preferred embodiment of the implantable medical device, the housing extends along an axial direction, wherein particularly the housing is an elongated housing with respect to the axial direction.

According to a further preferred embodiment of the implantable medical device, the connection member protrudes from a proximal end portion of the housing in the axial direction.

Furthermore, according to a preferred embodiment of the implantable medical device, the connection member comprises a head portion extending in a direction perpendicular to the axial direction of the housing, and a base portion integrally connecting the head portion to the housing, wherein the base portion protrudes from the proximal end portion of the housing in the axial direction.

According to yet another a preferred embodiment of the implantable medical device, the opening is at least partially formed in the head portion of the connection member.

Furthermore, according to a preferred embodiment of the implantable medical device, the implantable medical device is an implantable intracardiac or leadless pacemaker.

According to a further preferred embodiment of the implantable medical device, the implantable medical device comprises anchoring members protruding from a distal end portion of the housing for anchoring the implantable medical device in cardiac tissue.

Furthermore, according to a preferred embodiment of the implantable medical device, the implantable medical device comprises an electrode arranged on the proximal portion of the housing for contacting cardiac tissue when the anchoring members (e.g. tines) anchor the implantable medical device to the cardiac tissue.

According to a further preferred embodiment of the present invention, the metal is titanium or a titanium alloy. Preferably, in an embodiment, also the housing is formed out of titanium, e.g., titanium grade 1, or a titanium alloy.

Furthermore, according to a preferred embodiment of the implantable medical device, the housing is formed by at least one of: a non-additive or subtractive manufacturing process, particularly milling, cutting, or by injection-molding, stamping, or deep drawing..

According to a further aspect of the present invention, a method for forming a connection member on a housing of an implantable medical device, particularly of an implantable medical device according to the present invention, is disclosed, wherein the method comprises providing a housing of the implantable medical device and providing a layer of metal powder on an end portion of the housing, directing a laser beam onto the layer of metal powder to selectively melt the metal powder so as to form a layer of the connection member on the end portion of the housing when the melted powder solidifies, wherein the foregoing process is repeated multiple times by adding a new layer of metal powder on the preceding layer of the connection member and selectively melting the new layer of metal powder until the connection member is formed layer by layer in one piece on the end portion of the housing.

According to a preferred embodiment, providing the housing comprises at least one of: forming the housing by a non-additive manufacturing process, forming the housing by a subtractive manufacturing process, forming the housing at least partially by milling, forming the housing by casting, forming the housing at least partially by cutting, forming the housing at least partially by stamping. These individual processes can be combined. Particularly, after each such process, the housing may be sanded and/or polished and/or coated with a coating, particularly an insulating coating, for example a parylene coating.

Although the present invention has been described in various aspects and embodiments relating to a connection member (hitch) for a tether. Instead of such a connection member also other geometries on housings can be realized such as anchors, positioning aids for header cores/external wiring.

In the following, embodiments as well as further features and advantages of the present invention shall be described with reference to the Figures, wherein
- Fig. 1: shows a schematic view of the human heart;
- Fig. 2: a schematic view of an embodiment of an implantable medical device according to the present invention in form of an implantable leadless pacemaker;
- Fig. 3: shows a proximal end portion of the housing of an embodiment of an implantable medical device according to the present invention; and
- Fig. 4: shows an illustration of an embodiment of the method according to the present invention.

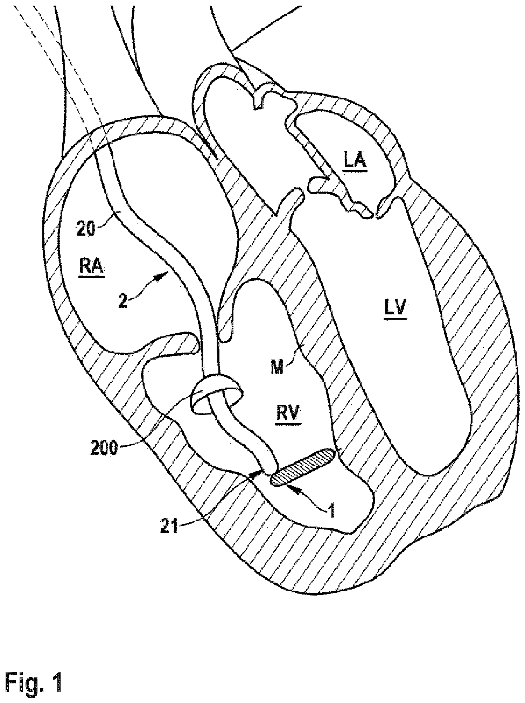

Fig. 1 shows, in a schematic drawing, a human heart comprising a right atrium RA, a right ventricle RV, a left atrium LA and a left ventricle LV, an implantable medical device 1 being implanted into the right ventricle RV, the implantable medical device 1 for example having the shape of an implantable leadless or intracardiac pacemaker 1 for providing a pacing of the heart's activity at the right ventricle RV.

For implantation the implantable medical device 1 is placed at an implant location by means of a delivery system 2, for example in the right ventricle RV - as illustrated in Fig. 1 - such that it comes to rest on myocardial tissue M.

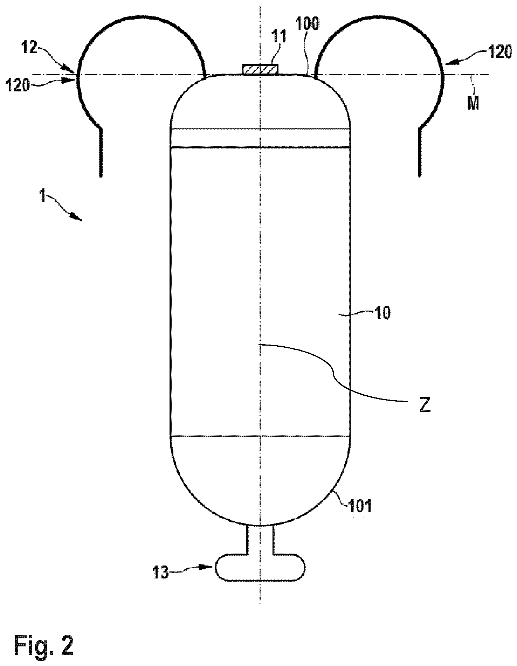

Fig. 2 shows, in a schematic drawing, an embodiment of an implantable medical device 1 according to the present invention in the form of an implantable leadless or intracardiac pacemaker 1 having a housing 10 forming a proximal end portion 101 and a distal end portion 100, the implantable medical device 1 being configured to be placed on myocardial tissue M by means of its distal end portion 100. On the distal end portion 100, an electrode 11 is arranged which, potentially together with other electrodes of the implantable medical device 1, serves to provide for a pacing action in order to stimulate cardiac activity in a defined manner.

During implantation, the implantable medical device 1 (here in form of an implantable leadless or intracardiac pacemaker 1) is to be placed at an implant location, for example on myocardial tissue M, and is to be fastened to tissue at the implant location. For this, the implantable medical device 1 comprises an anchoring device 12 having a multiplicity of anchoring members 120 in the shape of tines, wires, sheets or tubes or the like, the anchoring members 120 extending from the housing 10 of the implantable medical device 1 at the distal end portion 100 in order to engage with tissue for fastening the implantable medical device 1 to the tissue.

For implantation the implantable medical device 1 is to be delivered towards the implant location by means of a delivery system 2, the delivery system 2 comprising a delivery catheter 20 which is guided for example through the superior vena into the patient's heart to access, via the right atrium RA, the right ventricle RV. During delivery the implantable medical device 1 is fixed to the delivery catheter 20 and inserted in the end cap 200 but is to be released once the implantable medical device 1 has reached the implant location and is fixed to tissue in the region of the implant location.

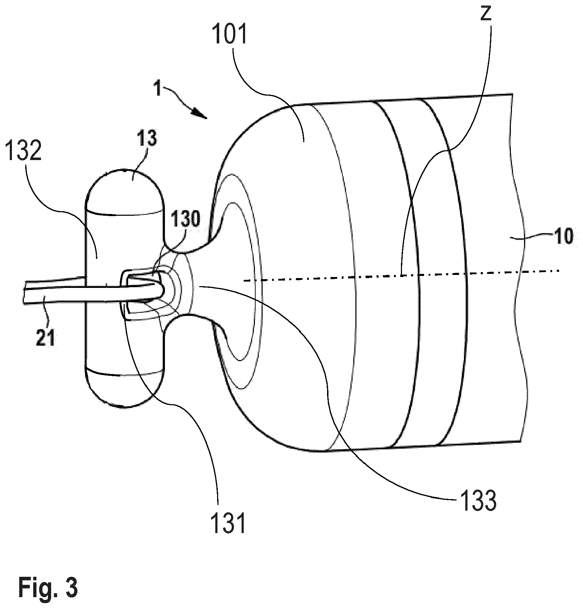

When the implantable medical device 1, by means of its distal end portion 100, is placed on tissue at the implant location, the anchoring members 120 of the anchoring device 12 come into engagement with the tissue such that the implantable medical device 1 is mechanically fixed to the tissue. Particularly, once the implantable medical device 1 is placed on the tissue at the implant location, a testing can be performed in order to ensure the mechanical fixation of the implantable medical device 1 to the tissue and in addition to test the proper functionality of the medical device 1, in particular for performing a pacing operation for example in the right ventricle RV of the patient's heart. In case the implantable medical device 1 is properly functioning and anchored, a tether 21 securing the device 1 to the delivery system 2 (cf. Fig. 3) can be released from a connection member 13 that is connected to the proximal portion 101.

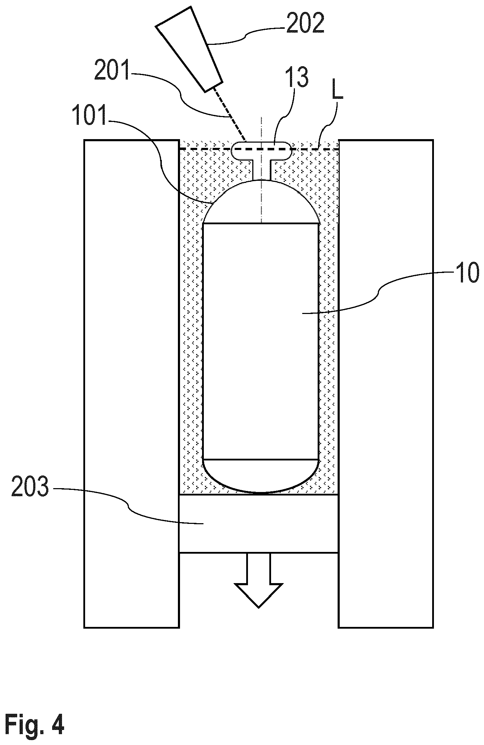

According to the present invention, the connection member 13 is formed on an outside of the housing 10, particularly on said proximal portion 101, out of a metal by selective laser melting (SLM) which is described further below regarding an embodiment of the method according to the present invention shown in Fig. 4.

Particularly, as shown in Fig. 3, the connection member 13 comprises an opening 130 through which the tether 21 extends when being connected to the connection member 13. Furthermore, the connection member 13 comprises a rounded surface 131 delimiting the opening 130 for contacting the tether 21 when the tether 21 is received in the opening 130 and extends along said surface 131. The rounded surface 131 helps to prevent that the tether is caught when moving through the opening 130. As also indicated in Fig. 3, the implantable medical device 1 preferably protrudes from the proximal end portion 101 of the housing 10 in the axial direction z and particularly comprises a head portion 132 extending in a direction perpendicular to the axial direction z, and a base portion 133 integrally connecting the head portion 132 to the housing 10, wherein the base portion 133 protrudes from the proximal end portion 101 of the housing 10 in the axial direction z. Particularly, the opening 130 is at least partially formed in the head portion 130.

Furthermore, as shown in Fig. 4, the base portion 133 and the head portion 132 as well as the opening 130 are formed on the end portion 101 of the housing 10 by way of selective laser melting (SLM). For this, the finished housing 10 without the connection member 13 is provided and a layer L of metal powder is arranged on the (e.g. proximal) end portion 101 of the housing 10. Then a laser beam 201 is directed by a laser 202 onto the layer L of metal powder to selectively melt the metal powder so as to form a layer of the connection member 13 on the end portion 101 of the housing 10 when the melted powder solidifies, wherein the foregoing process is repeated multiple times by adding a new layer L of metal powder on the preceding layer of the connection member 13 and selectively melting the new layer L of metal powder until the connection member 13 is formed layer by layer in one piece on the end portion 101 of the housing 10. The housing 10 with layers of the connection member 13 arranged thereon can be lowered for providing a new layer of metal powder on the existing part of the connection member 13 by lowering a build platform 203 on which the housing 10, said existing part of the connection member 13, and the metal powder are resting.

The present invention achieves a cost-saving assembly of the geometrically complicated connection member (hitch) on the inexpensively manufactured and chemically/biologically safe housing of an implantable medical device, particularly iLP. A significant reduction of the manufacturing costs of the (iLP) housing and possibly other housings and components machined with this process can be generated.

## Claims

1. An implantable medical device, comprising
- a housing (10) for accommodating at least one component of the implantable medical device (1), and
- a connection member (13) connected to the housing (10) and protruding from an outside of the housing (10),
wherein,
the connection member (13) is formed on an outside of the housing (10) out of a metal by selective laser melting.

2. The implantable medical device according to claim 1, wherein the connection member (13) is configured to be connected to a tether (21).

3. The implantable medical device according to claim 1 or 2, wherein the connection member (13) comprises an opening (130) through which the tether (21) extends when being connected to the connection member (13).

4. The implantable medical device according to one of the preceding claims, wherein the connection member (13) comprises a rounded surface (131) delimiting the opening (130) for contacting the tether (21) when the tether (21) is received in the opening (130) and extends along said surface (131).

5. The implantable medical device according to one of the preceding claims, wherein the housing (10) extends along an axial direction (z).

6. The implantable medical device according to claim 5, wherein the connection member (13) protrudes from a proximal end portion (101) of the housing (10) in the axial direction (z).

7. The implantable medical device according to claim 6, wherein the connection member (13) comprises a head portion (132) extending in a direction perpendicular to the axial direction (z) and a base portion (133) integrally connecting the head portion (132) to the housing (10), wherein the base portion (133) protrudes from the proximal end portion (101) of the housing (10) in the axial direction (z).

8. The implantable medical device according to claims 3 and 7, wherein the opening (130) is at least partially formed in the head portion (130).

9. The implantable medical device according to one of the preceding claims, wherein the implantable medical device (1) is an implantable inctracardiac pacemaker.

10. The implantable medical device according to one of the preceding claims, wherein the implantable medical device (1) comprises an anchoring device (12)) having one or more anchoring members (120), particularly tines, protruding from a distal end portion (100) of the housing (10) for anchoring the implantable medical device (1) in cardiac tissue.

11. The implantable medical device according to claim 10, wherein the implantable medical device (1) comprises an electrode (11) arranged on the proximal portion (100) of the housing (10) for contacting cardiac tissue when the anchoring device (12) anchor the implantable medical device (1) to the cardiac tissue.

12. The implantable medical device according to one of the preceding claims, wherein the metal is titanium or a titanium alloy.

13. The implantable medical device according to one of the preceding claims, wherein the housing is formed by at least one of: a non-additive manufacturing process, particularly milling, cutting, or by injection-molding, stamping, or deep drawing.

14. A method for forming a connection member (13) on a housing (10) of an implantable medical device (1), particularly of an implantable medical device (1) according to one of the preceding claims, wherein the method comprises: providing a housing (10) of the implantable medical device (1) and providing a layer (L) of metal powder on an end portion (101) of the housing (10), directing a laser beam (201) onto the layer (L) of metal powder to selectively melt the metal powder so as to form a layer of the connection member (13) on the end portion (101) of the housing (10) when the melted powder solidifies, wherein the foregoing process is repeated multiple times by adding a new layer (L) of metal powder on the preceding layer of the connection member (13) and selectively melting the new layer (L) of metal powder until the connection member (13) is formed layer by layer in one piece on the end portion (101) of the housing (10).

15. The method according to claim 14, wherein the housing (10) is provided by at least one of: a non-additive manufacturing process, particularly milling, cutting, or by injection-molding, stamping, or deep drawing.
